# EUROPEAN PATENT APPLICATION

(11) **EP 2 111 890 A1**
(43) Date of publication of application: **28.10.2009**
(21) Application number: 08706525.6
(22) Date of filing: 15.02.2008
(51) Int. Cl.: A61N 1/36, A61N 1/372

(54) **NON-ELECTRODE-LEAD ULTRA-THIN MICRO MULTIFUNCTIONAL HEART RATE ADJUSTING DEVICE**

(30) Priority: 16.02.2007 CN 200710037687
(71) Applicant: Sun Medical-Scientific (Shanghai) CO., LTD., Shanghai 201318 (CN)
(72) Inventor: SEN, Luyi, Shanghai 201201 (CN)
(74) Representative: Brown, David Leslie
(86) International application number: PCT/CN2008/000350
(87) International publication number: WO 2008/101409

(57) **Abstract**

A non-electrode-lead ultra-thin micro multifunctional heart rate adjusting device comprises an integrative ultra-thin micro non-electrode-lead pacemaker formed by assembling a micro battery, an ultra-low-power source circuit, a wireless receiving/transmitting circuit and an application circuit unit together, the needle electrodes are positioned on one side of the pacemaker and all of them form a small electrode body which can directly implanted into heart or external surface of heart; a multifunctional microcomputer heart rate adjusting remote controller connects with various non-electrode-lead pacemakers via wireless communication; the non-electrode-lead pacemakers and/or the heart rate adjusting remote controller connect with a control base station via wireless network, and the control base station connects with a computer.

## Description

### Cross Reference of Related Application

The present invention requests a priority of a China invention patent application, application No. 200710037687.6, filed Feb. 16, 2007.

### Background of the Present Invention

### Field of Invention

The present invention relates to a medical device, and more particularly to a medical device for regulating cardiac rhythm.

### Description of Related Arts

Pacemakers have been used for more than half a century, accompanied with the development of pacing mode from in vitro at the beginning to in vivo nowadays.

The external pacemaker, having a large size, is capable of replacing batteries at any moment and adjusting the pacing frequency, but it is not convenient to carry and is prone to infect at an entrance of a body electrode lead. As a result, the external pacemaker is clinically used for temporary pacing at present.

The implanted pacemaker is implanted under the skin, and has a need for surgery. It can be applied to permanent pacing on account of conveniently carrying and avoiding infection. However, it is necessary to replace the whole pacemaker by operation when the built-in batteries are used up.

Both the implanted pacemaker and the external pacemaker need one or more than one electrode leads to connect the pacing electrodes implanted into heart muscles with the pacing pulse generator.

The minimum pacemaker up to now has a size of 6×33×33mm and a weight of 12.8g, so it is impossible to be implanted directly into the heart.

The pacemaker with a large size has a restriction on contraction and relaxation of the heart as a result of the heart beating. Furthermore, it is not capable of fixing on the beating heart owing to the large size. If the pacemaker is sutured to the heart by force, the heart muscles are torn. Accordingly, the small electrodes are implanted into the endocardium and epicardium, and then connected with the pacemaker by electrode leads. The electrode lead, having a length of more than 10cm, passes through blood vessels to the heart so that thrombi are formed in the heart and blood vessels in general. If pacing happens in the ventricles, it is necessary for the electrode leads to pass through the tricuspid so that the tricuspid is not capable of closing completely. In addition, the permanent pacemaker implanted under the skin often leads to inductive contractions and vibrations of muscles all around, so that patients easily feel very uncomfortable.

As a temporary pacemaker, the electrode leads are drawn out of the skin and connected with the pacemaker in vitro. The wound is often injected at an entrance of a body electrode lead. Furthermore, the electrode leads of epicardium pacing pass through other organs and often cause adhesions.

Recently, a so-called wireless pacemaker is presented in a China application "Digital remote wireless electrocardiogram monitoring system", publication No. CN1657003A, published Aug. 24, 2005, which comprises two parts of a portable electrocardiogram signal acquisition, display and sending terminal, and a remote monitoring center. The terminal, having a size less than that of the palm, establishes a real-time connection with Internet by a digital cellular mobile communication network for sending collected electrocardiogram data of users to the remote monitoring center by Internet, and accepting the control of the remote monitoring center. The remote monitoring center receives, analyses, displays and storages the data by computers connected with Internet.

Nevertheless, the above mentioned technical solution means wireless control and information transmission of the implanted pacemaker with computers of the console. Accordingly, it is not the genuine non-electrode-lead pacemaker, and is not capable of resolving many defects from the existing electrode leads between the pacemaker and pacing electrodes.

### Summary of the Present Invention

A technology problem to be resolved is to provide a non-electrode-lead ultra-thin micro multifunctional heart rate adjusting device adapted for pacing, defibrillation and rhythm resynchronization, which integrates the existing pacemaker and pacing electrodes into one whole, and is capable of implanting directly into a corresponding position of endocardium or epicardium by intervention and/or minimal invasion to test corresponding bioelectrical signals.

The technology solution of the present invention is to provide a non-electrode-lead ultra-thin micro multifunctional heart rate adjusting device, which comprises a rhythm adjustment controller, a power source, a pacemaker and electrodes, wherein the power source, pacemaker and electrodes are provided within a human body,
wherein the power source, pacemaker and electrodes integrate into a whole as an ultra-thin micro non-electrode-lead pacemaker for regulating rhythm and defibrillating, formed by assembling an ultra-thin micro battery, an ultra-low-power source circuit, a wireless receiving/transmitting circuit and an application circuit unit together, which comprises a microcomputer rhythm adjusting system, wherein the ultra-thin micro non-electrode-lead pacemaker is foldably expanded, needle electrodes are provided at a side or end thereof, in such a manner that the ultra-thin micro non-electrode-lead pacemaker and the needle electrodes integrally form a small electrode body which is capable of implanting directly into a heart or exterior surface of the heart by intervention and/or minimal invasion;
wherein the ultra-thin micro non-electrode-lead pacemaker with the needle electrodes is provided at every position where the heart needs implanting;
wherein the rhythm adjustment controller comprises a multifunctional microcomputer rhythm adjustment remote controller, bering portable and implantable, which is corresponding to at least one or more ultra-thin micro non-electrode-lead pacemakers to transmit control signals and receive feedback signals thereof;
wherein the multifunctional microcomputer rhythm adjustment remote controller wirelessly communicates, synthetically regulates with the ultra-thin micro non-electrode-lead pacemaker to resynchronize rhythm;
wherein the ultra-thin micro non-electrode-lead pacemaker and/or multifunctional microcomputer rhythm adjustment remote controller connects with a controlling base station by wireless network, the controlling base station connects with a computer;
wherein the ultra-thin micro non-electrode-lead pacemaker further comprises a non-contact charging receiving circuit, the multifunctional microcomputer rhythm adjustment remote controller further comprises a non-contact charging receiving and/or transmitting circuit;
wherein the non-contact charging receiving circuit of the ultra-thin micro non-electrode-lead pacemaker is adapted for receiving charging from exterior non-contact charger, the non-contact charging receiving circuit of the multifunctional microcomputer rhythm adjustment remote controller is adapted for receiving charging from exterior non-contact charger, the non-contact charging transmitting circuit of the multifunctional microcomputer rhythm adjustment remote controller is adapted for charging the ultra-thin micro non-electrode-lead pacemaker;
wherein, when the multifunctional microcomputer rhythm adjustment remote controller is implanted into the human body, the multifunctional microcomputer rhythm adjustment remote controller acts as a intermediate charger not only for receiving charging of the external non-contact charger, but also for charging the ultra-thin micro non-electrode-lead pacemaker, so as to prolong service life of batteries provided therein.

Specially, the needle electrodes comprises a pacing electrode, a biosensor electrode coaxially aligned with the pacing electrode, wherein the pacing electrode and biosensor electrode are provided with multi core and multi layer, two insulating layers are provided between the pacing electrode and the biosensor electrode and provide at a peripheral edge of the biosensor electrode, respectively, wherein a whole length of the pacing electrode is larger than that of the biosensor electrode, wherein an end of the pacing electrode and biosensor electrode forms a biosensor or pulse output electrode port correspondingly connecting with the application circuit unit provided on a flexible line band or circuit board.

The ultra-thin micro-battery, ultra-low-consumption power circuit, wireless receiver/transmitter circuit, non-contact charging receiving circuit and application circuit unit are connected with each other by a flexible line band or circuit board, which can be foldedly expanded, wherein an anastomosed piece is provided at a peripheral edge of the ultra-thin micro non-electrode-lead pacemaker, four fastening pinholes are provided on the anastomosed piece to suture fixation and/or avoid falling off.

The ultra-thin micro-battery is a rechargeable battery or permanent battery.

Specially, the application circuit unit comprises a signal isolation and matching circuit, a biosensor receiver, a micro data processing data programming circuit, an oscillating and time control circuit, a first switching circuit, a converter circuit, a state and data record and control circuit, a second switching circuit, a pulse generator circuit, and a bioelectrical monitoring system circuit;
wherein, an input terminal of the signal isolation and matching circuit is connected with an electrode or antenna, first switching circuit, and second switching circuit, the biosensor is connected with the signal isolation and matching circuit, and the biosensor receiver, a first output terminal of the first switching circuit is connected with the oscillating and time control circuit and the micro data processing data programming circuit by the biosensor receiver, a second output terminal of the first switching circuit is connected with the state data record and control circuit by the converter circuit, an output terminal of the state data record and control circuit is connected with the wireless receiving/transmitting circuit, a first output terminal of the second switching circuit is connected with the wireless receiving/transmitting circuit by the pulse generator circuit, a second output terminal of the second switching circuit is connected with the wireless receiving/transmitting circuit by the bioelectrical monitoring system circuit.

Specially, the multifunctional microcomputer rhythm adjustment remote controller comprises a microcomputer circuit module connected by digital communication buses and an input/output circuit module, wherein the microcomputer circuit module comprises a microprocessor, a system clock, a RAM, a ROM, and a RAM/ROM control unit, wherein the input/output circuit module comprises a memory, a programming/time control/digital control unit, a A/D converter/detector unit, a sensing/filtering amplifier circuit, an electrode configuration exchanging circuit, a telemetry circuit/receiver/RF converting unit, a battery supply charging/ processing system, a voltage/current reference generator, a monitoring/ measuring system multiplexer ADC, and a wireless receiving/transmitting circuit;
wherein the programming/time control/digital control unit is connected with the memory and the telemetry circuit/receiver/RF converting unit, and connected with the sensing/filtering amplifier circuit and the electrode configuration exchanging circuit by the A/D converter/detector unit, the electrode configuration exchanging circuit is connected with the wireless receiving/transmitting circuit to bidirectionally exchanges signals, the battery supply charging/processing system is connected with the programming/time control/digital control unit, the monitoring/ measuring system multiplexer ADC and the wireless receiving/transmitting circuit by the voltage/current reference generator.

Specially, the controlling base station comprises a wake-up circuit/RF switching circuit/BSM module, a wireless signal receiving/transmitting circuit module, and a data interface/applied microcontroller/microprocessor/ADP module, wherein connecting, exchanging data and transferring instructions are carried out between the controlling base station and computer by wired or wireless manner.

The ultra-thin micro non-electrode-lead pacemaker receives control signals of the multifunctional microcomputer rhythm adjustment remote controller or controlling base station, and feeds back physiological electrical signals of every position where the ultra-thin micro non-electrode-lead pacemaker is provided; the multifunctional microcomputer rhythm adjustment remote controller or controlling base station takes charge of controlling operation of the ultra-thin micro non-electrode-lead pacemaker so as to control pacing, defibrillation and rhythm resynchronization thereof.

The multifunctional microcomputer rhythm adjustment remote controller communicates with the controlling base station by wireless network, receives control instructions, feeds back various physiological parameters and indexes of monitored objects, and adjusts working states of the ultra-thin micro non-electrode-lead pacemaker according to preset data.

Compared with the prior art, the present invention has several advantages of:
1. No electrode leads are provided between the pacemaker and the pacing electrode to avoid various shortcomings of existing technology;
2. The whole pacemaker has a small size, and is flexible, foldable. Single foldable ultra-thin micro non-electrode-lead pacemaker can be implanted by interventional catheter, or by thoracoscope or minimally invasive thoracotomy to not only reduce operation workload and patients' operation pains, but greatly decrease related cost of implanted pacemaker;
3. Non-contact charging is applied to greatly prolong working life of the device implanted into the human body, decrease fault rate, and avoid patient's operation pain and high cost;
4. The multifunctional microcomputer rhythm adjustment remote controller acts as a relay/transfer station of wireless signals, which itself has the same or similar control function as the controlling base station and computer, so the transmit power, received power and power consumption what the pacemaker implanted into the body needs are decreased greatly, to prolong the working time and life of the power thereof, also enlarge the range of patient's motion, and reduce influences of wireless signals on patients' healthy;
5. The whole rhythm adjustment device has more functions such as pacing, defibrillation, rhythm resynchronization and so on to satisfy higher medical/treatment requirement, which is more reliable and practical;
6. A new concept of rhythm adjustment without electrode leads is provided. The concept is applied to invent various micro non-electrode-lead single or multiple functional rhythm adjusting and control devices.

### Brief Description of the Drawings

Fig. 1 is a structural diagram of an existing pacemaker and shows an outline dimension thereof.
Fig. 2 is a structural diagram of a position at which an ultra-thin micro non-electrode-lead pacemaker is provided according to a preferred embodiment of the present invention and shows an outline dimension thereof.
Fig. 3 is a schematic diagram of the ultra-thin micro non-electrode-lead pacemaker provided at a corresponding position of the endocardium according to the above preferred embodiment of the present invention.
Fig. 4 is a schematic diagram of the ultra-thin micro non-electrode-lead pacemaker provided at a corresponding position of the epicardium according to the above preferred embodiment of the present invention.
Fig. 5 is a side view of the ultra-thin micro non-electrode-lead pacemaker according to the above preferred embodiment of the present invention.
Fig. 6 is a top view of the ultra-thin micro non-electrode-lead pacemaker according to the above preferred embodiment of the present invention.
Fig. 7 is partially enlarged view of biosensor or pulse output needle electrodes of the pacemaker.
Fig. 8 is a block diagram of a whole system of the present invention.
Fig. 9 is a block diagram based on the electrical principle of the implanted non-electrode-lead pacemaker.
Fig. 10 is a circuit diagram of the sensor with low power consumption.
Fig. 11 is a block diagram of multifunctional microcomputer rhythm adjustment remote controller.
Fig. 12 is a block diagram of a physiological signal micro-processing circuit with ultra-low consumption.
Fig. 13 is a circuit diagram of an electrode configuration exchanging circuit.
Fig. 14 is a block diagram of a battery charging system with low consumption.
Figs. 15-20 show several kinds of data flows, controlling signal flows and controlling means of the present invention.

### Detailed Description of the Preferred Embodiment

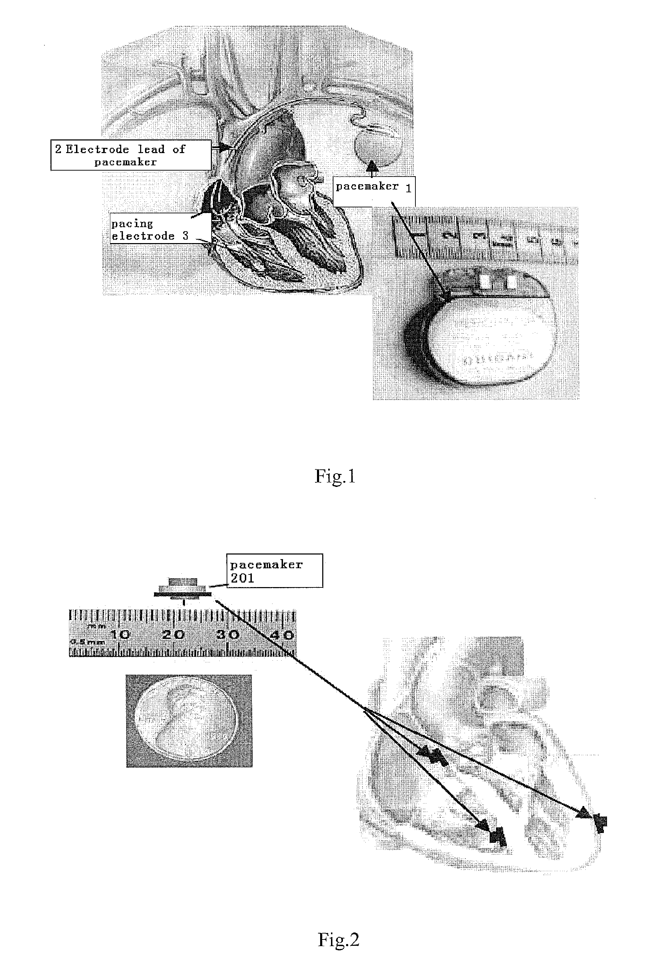

Referring to Fig. 1, the existing pacemaker, whether in vivo or in vitro, needs one or more electrode leads 2 to connect pacing electrodes 3 implanted into heart muscles with a pacing pulse generator 1.

The conventional minimum pacemaker has a size of 6×33×33mm, a weight of 12.8g up to now. Obviously, it is not capable of implanting directly into the heart.

As shown in Fig. 2, the present invention integrates a power supply, a pacemaker and electrodes into a whole as an ultra-thin micro non-electrode-lead pacemaker for regulating rhythm and defibrillating, formed by assembling an ultra-thin micro battery, an ultra-low-power source circuit, a wireless receiving/transmitting circuit and an application circuit unit together, which comprises a microcomputer rhythm adjusting system, wherein the ultra-thin micro non-electrode-lead pacemaker is foldably expanded, needle electrodes are provided at a side or end thereof, in such a manner that the ultra-thin micro non-electrode-lead pacemaker and the needle electrodes integrally form a small electrode body which is capable of implanting directly into a heart or exterior surface of the heart by intervention and/or minimal invasion.

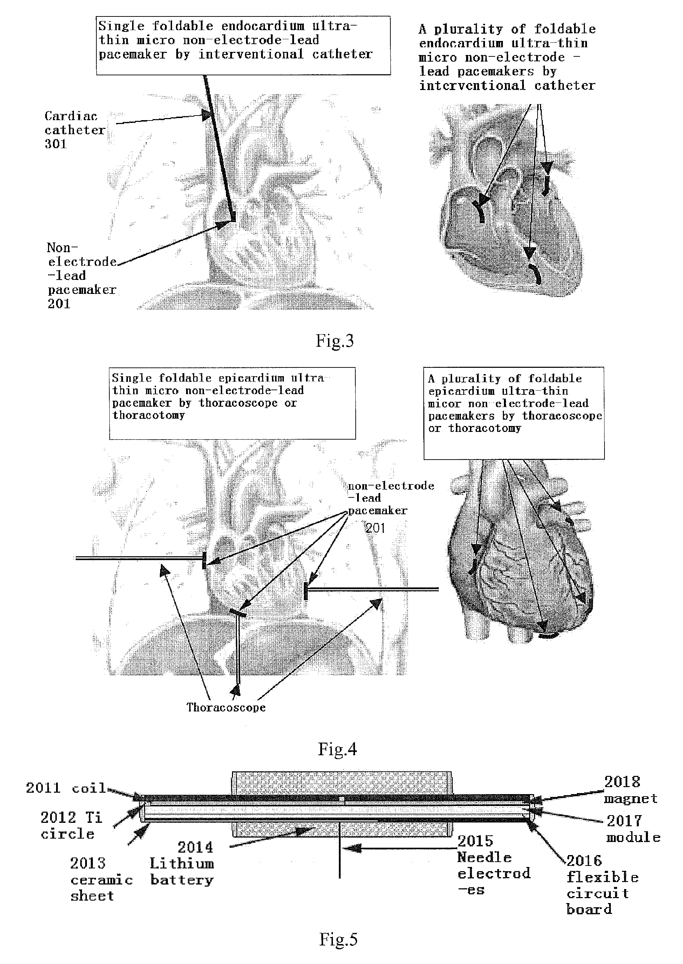

Fig. 3 is a schematic diagram of the ultra-thin micro non-electrode-lead pacemaker provided at a corresponding position of the endocardium according to the above preferred embodiment of the present invention. An ultra-thin micro non-electrode-lead pacemaker is implanted as shown in a left side of Fig. 3, and a plurality of ultra-thin micro non-electrode-lead pacemakers are implanted as shown in a right side of Fig. 3.

Because the ultra-thin micro non-electrode-lead pacemaker has a small size, an ultra-thin micro non-electrode-lead pacemaker with needle electrodes can be implanted into every position where the human body needs.

As shown in Fig. 3, the ultra-thin micro non-electrode-lead pacemaker is implanted at a needed position of endocardium.

The ultra-thin micro non-electrode-lead pacemaker is implanted at a needed position of epicardium in virtue of thoracoscope or thoracotomy, as shown in Fig. 4.

An ultra-thin micro non-electrode-lead pacemaker is implanted as shown in a left side of Fig. 4, and a plurality of ultra-thin micro non-electrode-lead pacemakers are implanted as shown in a right side of Fig. 4.

Accordingly, the ultra-thin micro non-electrode-lead pacemaker is implanted at a needed position of epicardium.

The rest are the same as Fig. 3.

Referring to Fig. 5, the present invention integrates a power supply, a pacemaker and electrodes into a whole as an ultra-thin micro non-electrode-lead pacemaker, which is foldably expanded. The ultra-thin micro non-electrode-lead pacemaker comprises an ultra-thin micro-battery (which is shown as a lithium battery 2014 in Figs), an ultra-low-consumption power circuit, a wireless receiver/transmitter circuit, a non-contact charging receiving circuit and an application circuit unit (all called as module 2017 in Figs), wherein needle electrodes 2015 are provided at a side or end thereof, the ultra-thin micro-battery, ultra-low-consumption power circuit, wireless receiver/transmitter circuit, non-contact charging receiving circuit and application circuit unit are connected with each other by flexible line band or circuit board to fold, expand or crimp.

A wireless transmitting/receiving antenna comprises a magnet 2018, a Ti circle (coil-skeleton) 2012, and a coil 2011 encircling the Ti circle.

All detecting, controlling circuits (as the above-mentioned ultra-low-consumption power circuit, wireless receiver/transmitter circuit, non-contact charging receiving circuit and application circuit unit, and so on) are provided on the flexible circuit board 2016, a ceramic sheet 2013 forms a shielding layer to prevent working signals from interfering the detection and signal output of the needle electrodes 2015.

The ultra-thin micro-battery is a rechargeable battery or permanent battery.

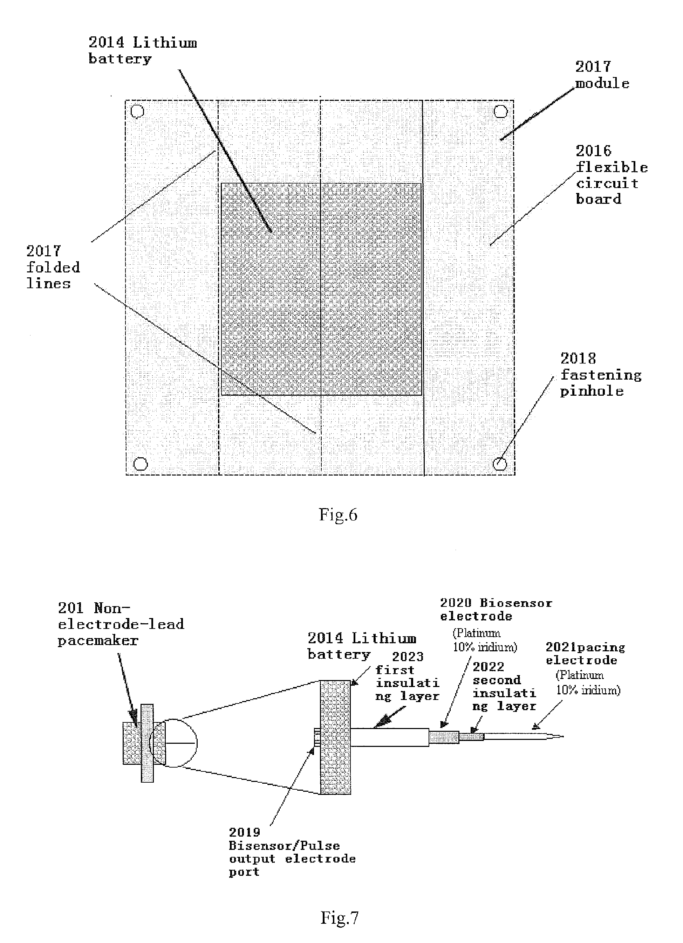

Referring to Fig. 6, two lithium batteries (also can be four, six and so on, the key is the number of the lithium batteries is even) are provided symmetrical longitudinally in the middle of the ultra-thin micro non-electrode-lead pacemaker. Two or four folded lines 2017 are provided at the whole flexible circuit board 2016. The flexible circuit board, with an area which is larger than a transverse sectional area of the lithium batteries, not only loads and connects relative circuits, but also acts as an anastomosed piece. Four fastening pinholes 201 are provided at four corners of the flexible circuit board respectively to suture fixation and/or avoid falling off.

On account of the above structure, the whole ultra-thin micro non-electrode-lead pacemaker is capable of folding and expanding so as to smoothly intervene the pipe or thoracoscope by folding or crimping.

As a result, the whole ultra-thin micro non-electrode-lead pacemaker is foldable or expandable to be folded or crimped into interventional catheter or thoracoscopy.

Thereby, 1) the interventional implantation is easily realized; 2) it provides sufficient flexibility and scalability for adapting to contraction and relaxation of the heart.

The present invention provides different ultra-thin micro non-electrode-lead pacemakers adapted for different occasions and functions, such as 1) endocardial pacing; 2) epicardium permanent pacing; and 3) epicardium temporary pacing.

A key of the above mentioned pacemaker with a reduced volume is that the volume of batteries is decreased. Presently, a volume of the smallest rechargeable battery is 100 times smaller than that of AA, but the smallest rechargeable battery is round-bar shaped. The smallest flat-shaped battery has a thickness of 1.7mm, a diameter of 17mm. Thereby, the volume of the rechargeable battery can be further decreased to a value that the thickness is 1.2mm, and the diameter is 8mm.

Furthermore, the inventor also designs another non-charging battery having a larger volume, which has a service life of more than ten years.

The ultra-thin micro non-electrode-lead pacemaker has three critical technology revolutions as follows: 1) an ultra-thin micro permanent or rechargeable battery; 2) an ultra-micro pulse generator; and 3) an ultra-micro computer pulse adjustor. Accordingly, the three parts integrates into a small pacemaker capable of implanting directly into the heart or clinging to epicardium.

Referring to Fig. 7, the needle electrodes, provided at a side or end of the ultra-thin micro non-electrode-lead pacemaker, comprises a pacing electrode 2021, a biosensor electrode 2020 coaxially aligned with the pacing electrode 2021, wherein the pacing electrode and biosensor electrode are provided with multi core or multi layer, a first insulating layer 2023 is provided at a peripheral edge of the biosensor electrode, and a second insulating layer 2022 is provided between the pacing electrode and the biosensor electrode, wherein a length of the pacing electrode is larger than that of the biosensor electrode. An end of the pacing electrode and biosensor electrode penetrates through a lithium battery 2014 to form a biosensor/pulse output electrode port 2019, connecting with a corresponding application circuit unit provided on the flexible circuit board.

In practice, the needle electrodes are inserted into the heart muscle, combined with the anastomosed piece and fastening pinholes, so as to not only prevent the ultra-thin micro non-electrode-lead pacemaker from falling off, but also avoid producing false signals and unnecessary electric shock.

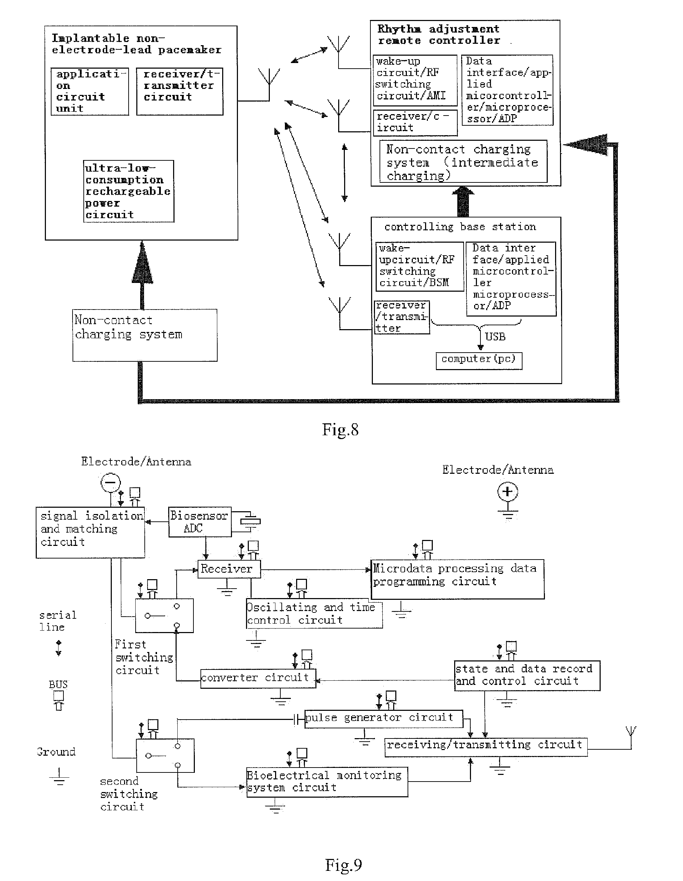

Fig. 8 is a block diagram composed of demonstrating circuits according to the present invention, showing three parts of an ultra-thin micro non-electrode-lead pacemaker, a rhythm adjustment remote controller and a controlling base station connecting with PC, wherein the ultra-thin micro non-electrode-lead pacemaker comprises an ultra-low-consumption power circuit, a wireless receiver/transmitter circuit, and an application circuit unit.

The rhythm adjustment remote controller comprises a multifunctional microcomputer rhythm adjustment remote controller which is portable or implantable, comprising a wake-up circuit/RF switching circuit/AMI module, and a data interface/applied microcontroller/microprocessor/ADP module.

The controlling base station comprises a wake-up circuit/RF switching circuit/BSM module, a wireless signal receiving/transmitting circuit module, and a data interface/ applied microcontroller/microprocessor/ADP module.

The ultra-thin micro non-electrode-lead pacemaker and/or the multifunctional microcomputer rhythm adjustment remote controller are/is connected with the controlling base station by wireless networks. Connecting, exchanging data and transferring instructions are carried out between the controlling base station and the computer by wired or wireless manner.

The multifunctional microcomputer rhythm adjustment remote controller corresponds to at least one (or a plurality of) ultra-thin micro non-electrode-lead pacemaker, gives control signals and receives return signals, synthetically regulates to resynchronize rhythm. The multifunctional microcomputer rhythm adjustment remote controller wirelessly communicates with the every ultra-thin micro non-electrode-lead pacemaker.

Considering the service life and volume of the battery supply, the ultra-thin micro non-electrode-lead pacemaker also can be provided with a non-contact charging receiving circuit, the multifunctional microcomputer rhythm adjustment remote controller also can be provided with a non-contact charging receiving and/or transmitting circuit.

The non-contact charging receiving circuit of the ultra-thin micro non-electrode-lead pacemaker is adapted for receiving the charge from exterior non-contact charger.

The non-contact charging receiving circuit of the multifunctional microcomputer rhythm adjustment remote controller is adapted for receiving the charge from exterior non-contact charger.

The non-contact charging transmitting circuit of the multifunctional microcomputer rhythm adjustment remote controller is adapted for charging the ultra-thin micro non-electrode-lead pacemaker.

Accordingly, the above mentioned design provides a convenience to the user. That is to say, the user is capable of charging the ultra-thin micro non-electrode-lead pacemaker and the multifunctional microcomputer rhythm adjustment remote controller by the external non-contact charging transmitting circuit, also is capable of firstly charging the multifunctional microcomputer rhythm adjustment remote controller, and then charging the ultra-thin micro non-electrode-lead pacemaker by the multifunctional microcomputer rhythm adjustment remote controller, so as to satisfy demands for different users or occasions.

As an application demonstration, when the multifunctional microcomputer rhythm adjustment remote controller implanted into the human body, it acts as an intermediate charger not only for receiving the charge of the external non-contact charger, but also for charging the ultra-thin micro non-electrode-lead pacemaker, so as to prolong service life of batteries provided within the ultra-thin micro non-electrode-lead pacemaker.

The external non-contact charging transmitting circuit can be provided separately, also can be provided with the controlling base station all together.

As a result of the modularized design, the above mentioned functions are easy to realize.

The technology, concrete circuitry, or working principle of the non-contact charging device can refer to a China invention patent called as "Non-contact charger", publication No.CN2891444A, or a China practical new patent called as "Non-contact charger for medical device in human body", publication No. CN2682716Y, no detailed description is provided herein.

The multifunctional microcomputer rhythm adjustment remote controller can be portable (such as mobile phone shaped, watch shaped), also can be implanted in vivo.

The multifunctional microcomputer rhythm adjustment remote controller has three main functions of: 1) pacing; 2) defibrillating; 3) cardiac resynchronization, but it also has a single function and dual functions. Considering the selection criteria of the multifunctional microcomputer rhythm adjustment remote controller, practical treatment need of a patent is necessary besides volume size. Furthermore, it is necessary to take into account the manufacturing cost. Accordingly, different function modules are chosen to achieve economical purchase and use cost on the basis of different customers.

The multifunctional microcomputer rhythm adjustment remote controller for cardiac resynchronization is multi-channel in general, because it is necessary for cardiac resynchronization to implant a plurality of ultra-thin micro non-electrode-lead pacemakers into the heart.

Multi-channel data transmission and control is an existing technology, no detailed description is provided herein.

Fig. 9 further illustrates the composition of the application circuit unit of the ultra-thin micro non-electrode-lead pacemaker, which at least comprises a signal isolation and matching circuit, a biosensor receiver, a micro data processing data programming circuit, an oscillating and time control circuit, a first switching circuit, a converter circuit, a state and data record and control circuit, a second switching circuit, a pulse generator circuit, and a bioelectrical monitoring system circuit;
wherein, an input terminal of the signal isolation and matching circuit is connected with the electrode or antenna, the first switching circuit, the second switching circuit, the biosensor is connected with the signal isolation and matching circuit, and the biosensor receiver, a first output terminal of the first switching circuit is connected with the oscillating and time control circuit and the micro data processing data programming circuit by the biosensor receiver, a second output terminal of the first switching circuit is connected with the state data record and control circuit by the converter circuit, an output terminal of the state data record and control circuit is connected with the wireless receiving/transmitting circuit, a first output terminal of the second switching circuit is connected with the wireless receiving/transmitting circuit by the pulse generator circuit, a second output terminal of the second switching circuit is connected with the wireless receiving/transmitting circuit by the bioelectrical monitoring system circuit.

It is worth to mention that the bioelectrical monitoring system circuit shown in Fig. 9 mainly functions in the defibrillation process.

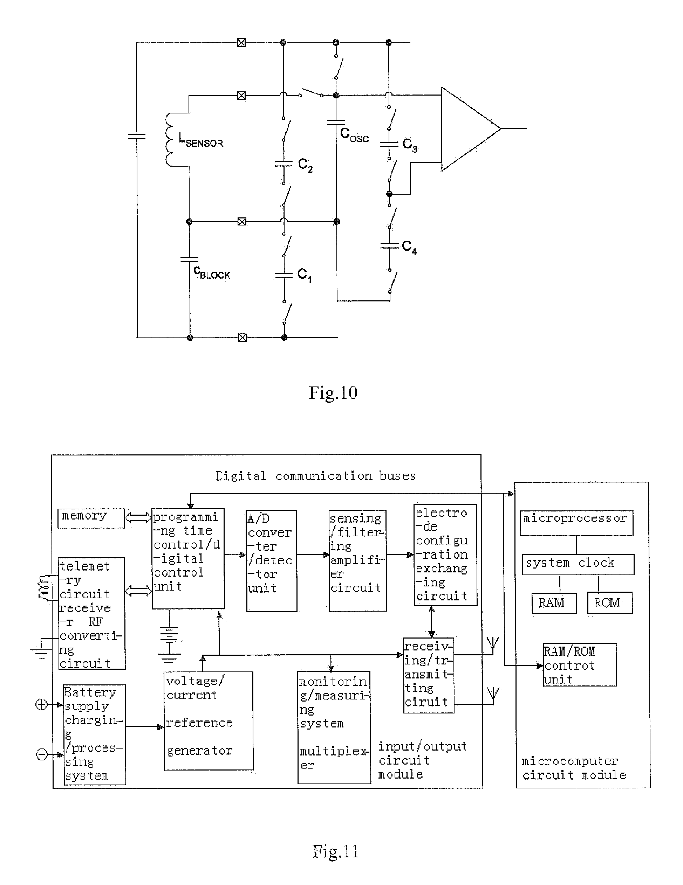

Fig. 10 further illustrates the low power consumption sensor circuit of the ultra-thin micro non-electrode-lead pacemaker. Under the control of the comparison circuit, according to different working states (or time sequences) of the sensor, every switch shown in Fig. 10 is turned on or off in sequence for greatly decreasing the electricity consumption of the whole sensor circuit so as to produce working currents only during the period of test demand and/or biological signal transmission to consume electric energy, and hardly electric energy is consumed in other time periods or time sequences.

Referring to the article of "Mixed-signal Integrated Circuits for Low Power, Battery Driven Applications" (http://www.techonline.com/learning/techpaper/197002893 or http://www.austriamicrosystems.com/02news/data/Austrochip2004_Villach_RForsyth.pdf), written by Austrian Richard M Forsyth, illustrating in detail the solution of designing a low-power mixed-signal circuit, optimizedly implementing all kinds of circuit modules, specific circuits and integrating wholly, no detailed description is provided herein.

Fig. 11 further illustrates an electric principle block diagram of the multifunctional microcomputer rhythm adjustment remote controller, which comprises a microcomputer circuit module connected by digital communication buses, and an input/output circuit module, wherein the microcomputer circuit module comprises a microprocessor, a system clock, a RAM, a ROM, and a RAM/ROM control unit, the input/output circuit module comprises a memory, a programming/time control/digital control unit, a A/D converter/detector unit, a sensing/filtering amplifier circuit, an electrode configuration exchanging circuit, a telemetry circuit/receiver/RF converting unit, a battery supply charging/processing system, a voltage/current reference generator, a monitoring/measuring system multiplexer ADC, and a wireless receiving/transmitting circuit;
wherein the programming/time control/digital control unit is connected with the memory and the telemetry circuit/receiver/RF converting unit, and connected with the sensing/filtering amplifier circuit and the electrode configuration exchanging circuit by the A/D converter/detector unit, the electrode configuration exchanging circuit is connected with the wireless receiving/transmitting circuit, and bidirectionally exchanges signals, the battery supply charging/processing system is connected with the programming/time control/digital control unit, the monitoring/measuring system multiplexer ADC and the wireless receiving/transmitting circuit by the voltage/current reference generator.

The voltage/current reference generator is more important and acts as an important role in the signal receiving and external charging process.

Fig. 12 concretely shows a block diagram of the physiological electrical signal micro-processing circuit with low power consumption, and further details the biosensor receiver module shown in Fig. 9.

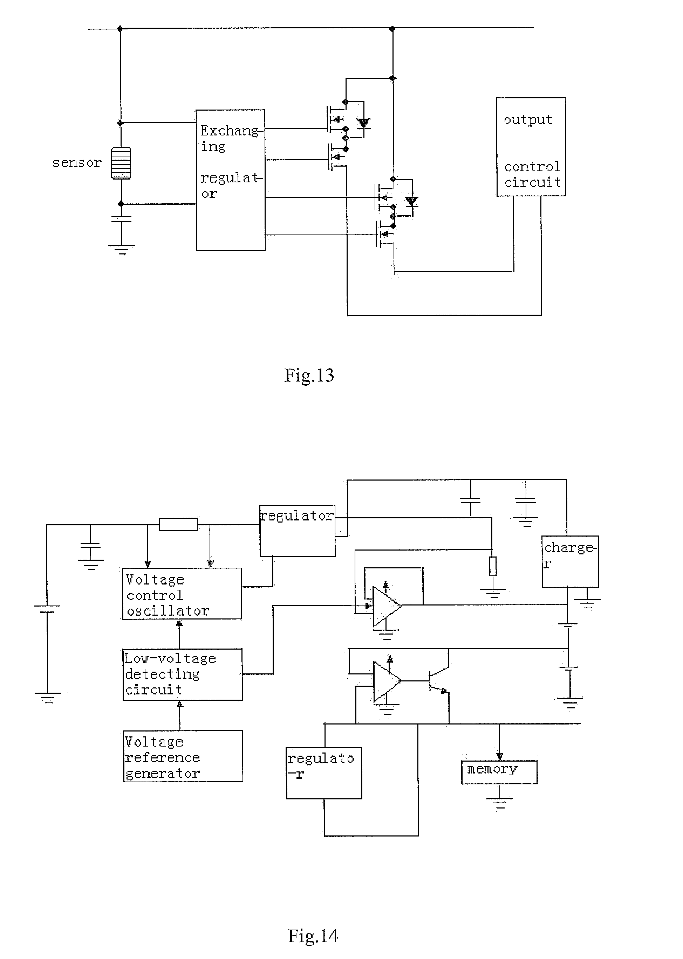

Fig. 13 concretely shows a block diagram of the electrode configuration exchanging circuit, and further details the electrode configuration exchanging circuit module shown in Fig. 10.

Fig. 14 concretely shows a block diagram of the battery supply charging processing system with low power consumption, and further details the ultra-low-power-consumption power circuit shown in Fig. 8.

Related contents of the above mentioned drawings are not exceed the range what one skilled in the art understands, so one skilled in the art will understand the meanings and design ideas of the present invention shown in the drawings, no detailed description is provided herein.

Referring to a China practical new patent called as "Cardiac electric artificial intelligence monitoring device of home computer", publication No.CN2569743Y, a China practical new patent called as "Remote real-time cardiac electric health care pre-diagnosis monitoring device", publication No.CN2836724Y, and a China practical new patent called as "Portable remote real-time monitor for high-risk heart disease patients", publication No. CN200977153Y, so as to have a better understanding of the above mentioned solution of the present invention.

Furthermore, referring to a PCT application called as "CIRCUIT AND METHOD FOR IMPLANTABLE DUAL SENSOR MEDICAL ELECTRICAL LEAD", international application NO. PCT/US99/24739, filed on 10/22/1999, priority date 10/22/1998, priority application NO. US 09/177,540, and a PCT application called as "LOW ENERGY PACING PULSE WAVEFORM FOR IMPLANTABLE PACEMAKER", international application NO. PCT/US1997/004840, filed on 25/03/1997, priority date 23/04/1996, priority application No. US 08/636,455.

It is worth to mention that every document described above is helpful to understand the present invention only and not intended to be limiting.

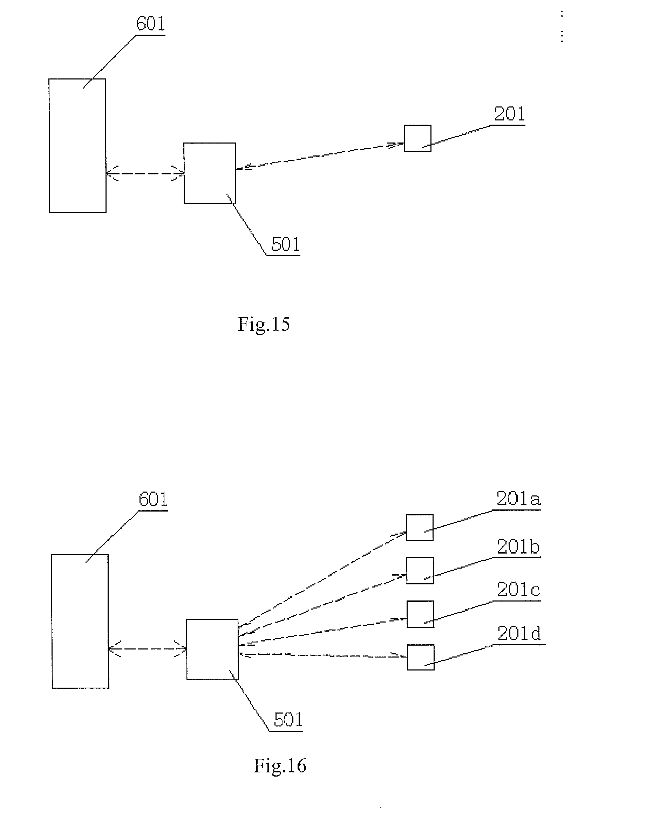

Referring to Figs. 15 to 20, several manners of exchanging data, controlling signal flow and control sequences of the whole system are provided, according to the present invention.

As shown in Fig. 15, the whole system comprises a non-electrode-lead pacemaker 201, a rhythm adjustment remote controller 501 and a controlling base station 601. The non-electrode-lead pacemaker receives control signals from the rhythm adjustment remote controller and feeds back physiological electrical signals of a position where the non-electrode-lead pacemaker is provided. The rhythm adjustment remote controller exchanges data and transmits control signals with the controlling base station. The rhythm adjustment remote controller receives control instructions from the controlling base station and feeds back various physiological parameters and indexes of monitored objects. Moreover, preset data is applied to adjust working states of the ultra-thin micro non-electrode-lead pacemaker so as to control pacing, defibrillation and rhythm resynchronization thereof.

Data and control signals are transferred between the non-electrode-lead pacemaker and the rhythm adjustment remote controller, between the rhythm adjustment remote controller and the controlling base station by wireless communication.

As shown in Fig. 16, the whole system comprises four non-electrode-lead pacemakers 201a-201d, a rhythm adjustment remote controller 501 and a controlling base station 601. Each of the non-electrode-lead pacemakers receives control signals from the rhythm adjustment remote controller and feeds back physiological electrical signals of a position where the every non-electrode-lead pacemaker is provided. The rhythm adjustment remote controller takes charge of controlling operation of each of the non-electrode-lead pacemakers. The rhythm adjustment remote controller exchanges data and transmits control signals with the controlling base station. The others are the same as shown in Fig. 15.

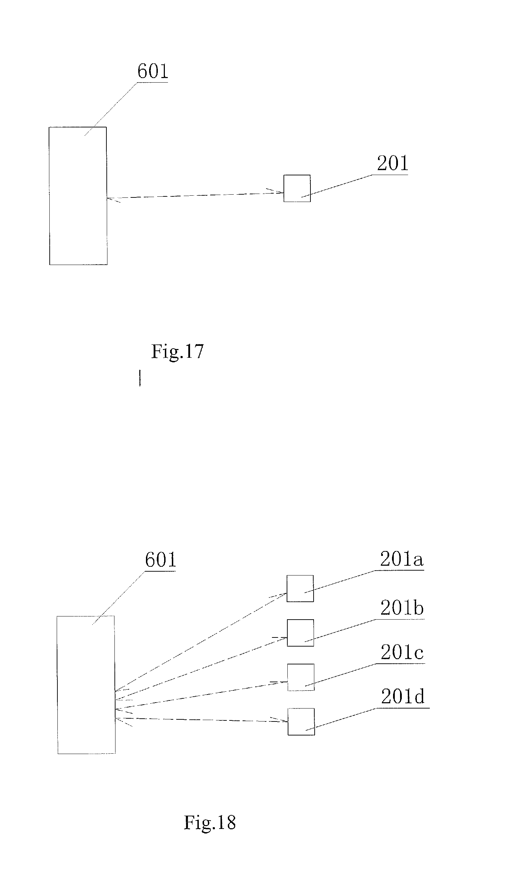

As shown in Fig. 17, the whole system comprises a non-electrode-lead pacemaker 201 and a controlling base station 601. The non-electrode-lead pacemaker directly receives control signals from the controlling base station and feeds back physiological electrical signals of a position where the non-electrode-lead pacemaker is provided. The controlling base station directly takes charge of controlling operation of the non-electrode-lead pacemaker, and adjusts working states of the non-electrode-lead pacemaker according to preset data so as to control pacing, defibrillation and rhythm resynchronization thereof.

As shown in Fig. 18, the whole system comprises four non-electrode-lead pacemakers 201a-201d and a controlling base station 601. Each of the non-electrode-lead pacemakers directly receives control signals from the controlling base station and feeds back physiological electrical signals of a position where the every non-electrode-lead pacemaker is provided. The controlling base station directly takes charge of controlling operation of each of the non-electrode-lead pacemakers. The others are the same as shown in Fig. 17.

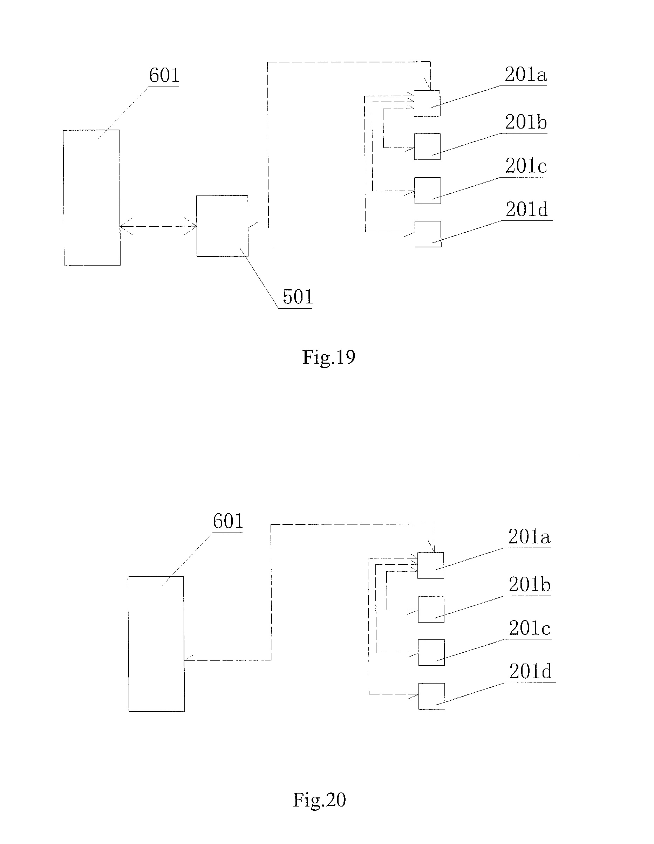

As shown in Fig. 19, the whole system comprises four non-electrode-lead pacemakers 201a-201d, a rhythm adjustment remote controller 501 and a controlling base station 601. The non-electrode-lead pacemaker 201a firstly receives control signals from the rhythm adjustment remote controller and then transmits the control signals to the non-electrode-lead pacemakers 201b-201d respectively. At the same time, the non-electrode-lead pacemaker 201a still receives the feedback physiological electrical signals of the non-electrode-lead pacemakers 201b-201d, and transmits to the rhythm adjustment remote controller all together, and then to the controlling base station by the rhythm adjustment remote controller. The rhythm adjustment remote controller exchanges data and transmits control signals with the non-electrode-lead pacemakers 201b-201d by the non-electrode-lead pacemaker 201a. Here, the non-electrode-lead pacemaker 201a acts as a relay and transfer station.

As shown in Fig. 20, the whole system comprises four non-electrode-lead pacemakers 201a-201d and a controlling base station 601. The non-electrode-lead pacemaker 201a firstly receives control signals from the controlling base station and then transmits the control signals to the non-electrode-lead pacemakers 201b-201d respectively. At the same time, the non-electrode-lead pacemaker 201a still receives the feedback physiological electrical signals of the non-electrode-lead pacemakers 201b-201d, and transmits to the controlling base station all together. The controlling base station exchanges data and transmits control signals with the non-electrode-lead pacemakers 201b-201d by the non-electrode-lead pacemaker 201a. Here, the non-electrode-lead pacemaker 201 a also acts as a relay and transfer station.

Obviously, every non-electrode-lead pacemaker receives control signals of the rhythm adjustment remote controller or controlling base station, and feeds back physiological electrical signals of a position where the every non-electrode-lead pacemaker is provided. And the rhythm adjustment remote controller or controlling base station takes charge of controlling operation of every non-electrode-lead pacemaker to control pacing, defibrillation and rhythm resynchronization thereof.

All in all, the present invention has creative breakthrough at four aspects as follows:
1. The ultra-thin micro non-electrode-lead pacemaker integrates electrodes, electrode leads and pacemaker as a whole, which includes three critical technology revolutions: 1) the ultra-thin micro permanent or rechargeable battery; 2) the ultra-micro pulse generator; and 3) ultra-micro computer pulse adjustor. Accordingly, the three parts are integrated into an electrode body which is capable of bending or folding, and implanting directly into the heart or clinging to epicardium by interventional and/or minimally invasive means.
2. Various designs of the ultra-micro wireless pulse generators with various applications, which are adapted for: 1) endocardial pacing; 2) epicardium permanent pacing; 3) epicardium temporary pacing.
3. Multi-channel wireless microcomputer rhythm adjustment remote controller has at least three main functions of: 1) pacing; 2) defibrillation; and 3) rhythm resynchronization.
4. Network connection and control of the wireless microcomputer rhythm adjustment remote controller: 1) transmitter; 2) receiver; and 3) control software.

The main function of the ultra-thin micro non-electrode-lead pacemaker (pacing/defibrillation/cardiac resynchronization) is to remain normal rhythm and strength cardiac systolic function.

The present invention can be widely applied in the therapeutic field for various patients with arrhythmias and systolic dysfunction.

It must be understood that the above described embodiment has been set forth only for the purposes of example and that it should not be taken as limiting invention as defined by the following invention and its various embodiments. Many alterations and modifications may be made by those having ordinary skill in the art without departing from the spirit and scope of the invention.

Although the above description illustrates the functions of every element (such as system, unit, module, circuit or member) in some combinations, it is definite understanding that one or more elements of requesting protection combinations can be deleted in some cases, the requesting protection combinations can be subcombinations or variants of subcombinations.

Especially consider that, such as one skilled in the art, non-substantial changes for the subject matter according to the existing technology or later design will still be in the scope of following claims. Accordingly, significant replacement one skilled in the art knows presently or later also belongs to the scope of following claims.

Thereby, the claims of the present invention includes all above drawing description, all equivalents on conception, and all obvious substitutes having primary ideas of the present invention.

One skilled in the art should know that this invention includes all modifications encompassed within the spirit and scope of the following claims.

### Industry Applications

The present invention integrates the existing pacemaker and pacing electrodes into one whole, without electrode leads. The pacemaker in the present invention, having a small size and foldable, is capable of implanted directly into the corresponding positions of the heart or epicardium by interventional and/or minimally invasive means, and testing corresponding bioelectrical signals. Furthermore, it has many functions such as pacing, defibrillation, rhythm resynchronization and so on. The pacemaker in the present invention can be charged non-contactly so that the working life of the device implanted into the body is prolonged greatly, and fault rate of the device implanted into the body is decreased. The multifunctional microcomputer rhythm adjustment remote controller acts as a relay or transfer station, so the transmit power, received power and power consumption what the pacemaker implanted into the body needs are decreased greatly, which is helpful to prolong the working time and life of the power thereof. The whole rhythm adjustment system, having more functions, can satisfy a higher medical/treatment requirement. Accordingly, it can be more reliable, practical, and widely used in the therapeutic field for various patients with arrhythmias and systolic dysfunction.

## Claims

1. A non-electrode-lead ultra-thin micro multifunctional heart rate adjusting device, comprising a rhythm adjustment controller, a power source, a pacemaker and electrodes, wherein said power source, pacemaker and electrodes are provided within a human body;
wherein said power source, pacemaker and electrodes integrate into a whole as an ultra-thin micro non-electrode-lead pacemaker for regulating rhythm and defibrillating, formed by assembling an ultra-thin micro battery, an ultra-low-power source circuit, a wireless receiving/transmitting circuit and an application circuit unit together, which comprises a microcomputer rhythm adjusting system, wherein said ultra-thin micro non-electrode-lead pacemaker is foldably expanded, needle electrodes are provided at a side or end thereof, in such a manner that said ultra-thin micro non-electrode-lead pacemaker and said needle electrodes integrally form a small electrode body which is capable of implanting directly into a heart or exterior surface of the heart by intervention and/or minimal invasion;
wherein said ultra-thin micro non-electrode-lead pacemaker with said needle electrodes is provided at every position where the heart needs implanting;
wherein said rhythm adjustment controller comprises a multifunctional microcomputer rhythm adjustment remote controller, portable and implantable, which is corresponding to at least one or more said ultra-thin micro non-electrode-lead pacemakers to transmit control signals and receive feedback signals thereof;
wherein said multifunctional microcomputer rhythm adjustment remote controller wirelessly communicates, synthetically regulates with said ultra-thin micro non-electrode-lead pacemaker to resynchronize rhythm;
wherein said ultra-thin micro non-electrode-lead pacemaker and/or said multifunctional microcomputer rhythm adjustment remote controller connect with a controlling base station by wireless network, said controlling base station connects with the computer.

2. The non-electrode-lead ultra-thin micro multifunctional heart rate adjusting device, as recited in claim 1, wherein said ultra-thin micro non-electrode-lead pacemaker further comprises a non-contact charging receiving circuit, said multifunctional microcomputer rhythm adjustment remote controller further comprises a non-contact charging receiving and/or transmitting circuit, said non-contact charging receiving circuit of said ultra-thin micro non-electrode-lead pacemaker is adapted for receiving charging from exterior non-contact charger, said non-contact charging receiving circuit of said multifunctional microcomputer rhythm adjustment remote controller is adapted for receiving charging from exterior non-contact charger, said non-contact charging transmitting circuit of said multifunctional microcomputer rhythm adjustment remote controller is adapted for charging said ultra-thin micro non-electrode-lead pacemaker;
wherein, when said multifunctional microcomputer rhythm adjustment remote controller is implanted into the human body, said multifunctional microcomputer rhythm adjustment remote controller acts as an intermediate charger not only for receiving charging of said external non-contact charger, but also for charging said ultra-thin micro non-electrode-lead pacemaker, so as to prolong service life of batteries provided within said ultra-thin micro non-electrode-lead pacemaker.

3. The non-electrode-lead ultra-thin micro multifunctional heart rate adjusting device, as recited in claim 1, wherein said needle electrodes comprises a pacing electrode, a biosensor electrode coaxially aligned with said pacing electrode, wherein said pacing electrode and biosensor electrode are provided with multi core or multi layer, two insulating layers are provided between said pacing electrode and said biosensor electrode and provide at a peripheral edge of said biosensor electrode respectively, wherein a whole length of said pacing electrode is larger than that of said biosensor electrode, wherein an end of said pacing electrode and biosensor electrode forms a biosensor or pulse output electrode port correspondingly connecting with said application circuit unit provided on a flexible line band or circuit board.

4. The non-electrode-lead ultra-thin micro multifunctional heart rate adjusting device, as recited in claim 1 or 2, wherein said ultra-thin micro-battery, ultra-low-consumption power circuit, wireless receiver/transmitter circuit, non-contact charging receiving circuit and application circuit unit are connected with each other by a flexible line band or circuit board to fold expand, wherein an anastomosed piece is provided at a peripheral edge of said ultra-thin micro non-electrode-lead pacemaker, four fastening pinholes are provided on said anastomosed piece to suture fixation and/or avoid falling off.

5. The non-electrode-lead ultra-thin micro multifunctional heart rate adjusting device, as recited in claim 1, wherein said ultra-thin micro-battery is a rechargeable battery or permanent battery.

6. The non-electrode-lead ultra-thin micro multifunctional heart rate adjusting device, as recited in claim 1, wherein said application circuit unit comprises a signal isolation and matching circuit, a biosensor receiver, a micro data processing data programming circuit, an oscillating and time control circuit, a first switching circuit, a converter circuit, a state and data record and control circuit, a second switching circuit, a pulse generator circuit, and a bioelectrical monitoring system circuit;
wherein, an input terminal of said signal isolation and matching circuit is connected with an electrode or antenna, first switching circuit, and second switching circuit, said biosensor is connected with said signal isolation and matching circuit, and said biosensor receiver, a first output terminal of said first switching circuit is connected with said oscillating and time control circuit and said micro data processing data programming circuit by said biosensor receiver, a second output terminal of said first switching circuit is connected with said state data record and control circuit by said converter circuit, an output terminal of said state data record and control circuit is connected with said wireless receiving/transmitting circuit, a first output terminal of said second switching circuit is connected with said wireless receiving/transmitting circuit by said pulse generator circuit, a second output terminal of said second switching circuit is connected with said wireless receiving/transmitting circuit by said bioelectrical monitoring system circuit.

7. The non-electrode-lead ultra-thin micro multifunctional heart rate adjusting device, as recited in claim 1, wherein said multifunctional microcomputer rhythm adjustment remote controller comprises a microcomputer circuit module connected by digital communication buses and an input/output circuit module, wherein said microcomputer circuit module comprises a microprocessor, a system clock, a RAM, a ROM, and a RAM/ROM control unit, wherein said input/output circuit module comprises a memory, a programming/time control/digital control unit, a A/D converter/detector unit, a sensing/filtering amplifier circuit, an electrode configuration exchanging circuit, a telemetry circuit/receiver/RF converting unit, a battery supply charging/ processing system, a voltage/current reference generator, a monitoring/ measuring system multiplexer ADC, and a wireless receiving/transmitting circuit;
wherein said programming/time control/digital control unit is connected with said memory and said telemetry circuit/receiver/RF converting unit, and connected with said sensing/filtering amplifier circuit and said electrode configuration exchanging circuit by said A/D converter/detector unit, said electrode configuration exchanging circuit is connected with said wireless receiving/transmitting circuit to bidirectionally exchanges signals, said battery supply charging/processing system is connected with said programming/time control/digital control unit, said monitoring/ measuring system multiplexer ADC and said wireless receiving/transmitting circuit by said voltage/current reference generator.

8. The non-electrode-lead ultra-thin micro multifunctional heart rate adjusting device, as recited in claim 1, wherein said controlling base station comprises a wake-up circuit/RF switching circuit/BSM module, a wireless signal receiving/transmitting circuit module, and a data interface/applied microcontroller/microprocessor/ADP module, wherein connecting, exchanging data and transferring instructions are carried out between said controlling base station and computer by wired or wireless manner.

9. The non-electrode-lead ultra-thin micro multifunctional heart rate adjusting device, as recited in claim 1, wherein said ultra-thin micro non-electrode-lead pacemaker receives control signals of said multifunctional microcomputer rhythm adjustment remote controller or controlling base station, and feeds back physiological electrical signals of every position where said ultra-thin micro non-electrode-lead pacemaker is provided; said multifunctional microcomputer rhythm adjustment remote controller or controlling base station take charge of controlling operation of said ultra-thin micro non-electrode-lead pacemaker to control pacing, defibrillation and rhythm resynchronization thereof.

10. The non-electrode-lead ultra-thin micro multifunctional heart rate adjusting device, as recited in claim 1, wherein said multifunctional microcomputer rhythm adjustment remote controller communicates with said controlling base station by wireless network, receives control instructions, feeds back various physiological parameters and indexes of monitored objects, and adjusts working states of said ultra-thin micro non-electrode-lead pacemaker according to preset data.
